# EUROPÄISCHE PATENTSCHRIFT

(11) **EP 1 232 145 B1**
(45) Veröffentlichungstag und Bekanntmachung des Hinweises auf die Patenterteilung: **02.07.2003**
(21) Anmeldenummer: 00972816.3
(22) Anmeldetag: 20.10.2000
(51) Int. Cl.: C07D 213/30

(54) **VERFAHREN ZUR HERSTELLUNG VON HYDROXYMETHYLPYRIDINEN**
METHOD FOR PRODUCING HYDROXYMETHYLPYRIDINES
PROCEDE DE PRODUCTION D'HYDROXYMETHYLPYRIDINES

(30) Priorität: 26.11.1999 AT 200499
(43) Veröffentlichungstag der Anmeldung: 21.08.2002
(73) Patentinhaber: DSM Fine Chemicals Austria Nfg GmbH & Co KG, 4021 Linz (AT)
(72) Erfinder: GISELBRECHT, Karlheinz, A-4061 Pasching (AT); HERMANSEDER, Rudolf, A-4624 Pennewang (AT)
(74) Vertreter: Lindinger, Ingrid
(86) Internationale Anmeldenummer: EP0010319
(87) Internationale Veröffentlichungsnummer: WO01038307

(56) Entgegenhaltungen:
- US-A- 3 637 721
- DATABASE CHEMABS [Online] CHEMICAL ABSTRACTS SERVICE, COLUMBUS, OHIO, US; AMANO, MASAAKI ET AL: "Preparation of hydroxymethylpyridine from cyanopyridine" retrieved from STN Database accession no. 127:293140 XP002158057 in der Anmeldung erwähnt & JP 09 255654 A (YUKI GOSEI KOGYO CO., LTD., JAPAN) 30. September 1997 (1997-09-30)
- QUEGUINER, GUY ET AL: "Pyridines. I. Diformylpyridines" BULL. SOC. CHIM. FR. (1968), (10), 4117-21 , XP002158055
- R.W. WHITE: "Catalytic Reduction of Ozonides. I. Synthesis of Alcohols from Olefinns " TETRAHEDRON LETTERS, Bd. 39, 1971, Seiten 3587-3589, XP002158056
- DATABASE CHEMABS [Online] CHEMICAL ABSTRACTS SERVICE, COLUMBUS, OHIO, US; SHIMANSKAYA, M. V. ET AL: "2-, 3-, Or 4-(hydroxymethyl)pyridines" retrieved from STN Database accession no. 103:215198 XP002158058 in der Anmeldung erwähnt & SU 1 167 182 A (INSTITUTE OF ORGANIC SYNTHESIS, ACADEMY OF SCIENCES, LATVIAN S.S.R., U) 15. Juli 1985 (1985-07-15)

## Beschreibung

Hydroxymethylpyridine sind wertvolle Zwischenprodukte und finden beispielsweise bei der Synthese von Pharma- und Agrarchemikalien. wie etwa für Mefioquine Hydrochlorid oder Pineprofen, Verwendung.

In der Literatur ist bereits eine Vielzahl von unterschiedlichsten Verfahrensvarianten für deren Herstellung beschrieben.

Aus Chem. Abstr. Vol.127: 293140, Vol.106: 18373 oder Vol.103: 215195 ist bekannt, dass 2- oder 4- Hydroxypyridin in einer Ausbeute von etwa 81 bis 87% durch katalytische Reduktion des entsprechenden 2-Cyanopyridins hergestellt werden können

Nachteilig bei diesem Verfahren sind der erforderliche hohe Druck und vor allem der hohe Preis des Ausgangsproduktes.

Eine weitere Möglichkeit ist die katalytische Reduktion von Pyridinaldehyden mit H₂ über Ba-promoted Cu-chromit Katalysatoren bei 180 bis 230°C gemäß Chem. Abstr. Vol. 103: 215198 oder die Reduktion mit NaBH₄ gemäß Chem. Abstr. Vol 93: 71487, wobei in Chem. Abstr. Vol 93: 71487 darauf hingewiesen wird, dass die Reaktivität der Aldehyde bei der Reduktion in der Reihenfolge 2,6-Pyridindicarboxaldehyd, 6-Methyl-2-Pyridincarboxaldehyd, 4- Pyridincarboxaldehyd, 2- Pyridincarboxaldehyd und 3- Pyridincarboxaldehyd abnimmt.

Der Nachteil dieser katalytischen Hydrierungen ist, das durch die Deaktivierung des jeweiligen Katalysators ein großer Anteil an Aldehyd nicht umgesetzt wird und das Endprodukt somit durch große Mengen an Aldehyd verunreinigt wird.

Ein weiterer Nachteil ist die schlechte Verfügbarkeit des Pyridinaldehyds.

Andere bekannte Varianten sind beispielsweise die elektrochemische Reduktion von Picolinsäureester (z.B.: EP-A1-0 189 678) oder die Oxidation von alpha-Picolin-N-Oxiden mit Trifluoroessigsäureanhydrid (z.B.: Chem. Abstr. Vol. 122: 239462)

Aufgabe der vorliegenden Erfindung war es ein Verfahren zur Herstellung von Hydroxymethylpyridinen zu finden, das von leicht zugänglichen Ausgangsverbindungen ausgeht und das eine hohe Reinheit der gewünschten Endprodukte gewährleistet, wobei störende Nebenprodukte auf einfachem Weg zu entfernen sind.

Gegenstand der Erfindung ist demnach ein Verfahren zur Herstellung von Hydroxymethylpyridinen, das dadurch gekennzeichnet ist, dass
a) das entsprechende Vinylpyridin in einem Alkohol als Lösungmittel ozonisiert und die dabei entstehende peroxidhaltige Lösung in Gegenwart eines Hydrierkatalysators hydriert wird,
b) die Hydrierlösung durch Zugabe einer Base auf einen pH-Wert von 10 bis14 gestellt und der Alkohol abdestilliert wird, worauf
c) das gewünschte Hydroxymethylpyridin durch Extraktion mit einem organischem Lösungsmittel und nachfolgender destillativer Aufreinigung in hoher Reinheit isoliert wird.

Durch das erfindungsgemäße Verfahren werden Hydroxymethylpyridine (HMPs) in hoher Reinheit hergestellt. Das Verfahren eignet sich dabei zur Herstellung einer Vielzahl Hydroxymethylpyridinen wie etwa von 2-, 3- oder 4-Hydroxymethylpyridin, 6-Methyl-2-hydroxymethylpyridin, 2,6-Di(Hydroxymethyl)-pyridin u.s.w. Bevorzugt werden 2-, 3- oder 4-Hydroxymethylpyridin, besonders bevorzugt 2-Hydroxymethylpyridin, erfindungsgemäß hergestellt.

Als Ausgangsverbindung dient das entsprechende Vinylpyridin, wie etwa 2-, 3- oder 4-Vinylpyridin, 6-Methyl-2-vinylpyridin und 2,6-Divinylpyridin, das im ersten Schritt mittels Ozonolyse mit nachfolgender katalytischer Hydrierung umgesetzt wird.

Die Ozonolyse wird bei Temperaturen von -80°C bis +20°C, bevorzugt bei -30°C bis +10°C und besonders bevorzugt bei -25°C bis +5°C, durchgeführt.

Das entsprechende Vinylpyridin wird mit maximal der äquivalenten Menge Ozon, umgesetzt, wobei unter den angegebenen Verfahrensbedingungen die Ozonolyse unter dem theoretischen Ozonverbrauch verläuft.

Es werden dabei stöchiometrische Mengen des jeweils eingesetzten Vinylpyridins verbraucht. Nach Beendigung der Ozonisierung sind keine Maßnahmen erforderlich, um überschüssiges oder nicht umgesetztes Ozon vor der Hydrierung aus dem Reaktionsgemisch zu vertreiben.

Die Umsetzung mit Ozon erfolgt in einem aliphatischen C₁-C₄-Alkohol, wie Methanol, Ethanol, Butanol oder Propanol. Bevorzugte Lösungsmittel sind Methanol und Ethanol, wobei Methanol wiederum besonders bevorzugt ist.

Die an die Ozonisierung anschließende katalytische Hydrierung der Ozonolyseprodukte erfolgt bevorzugt in stark verdünnter Lösung, wobei durch die im folgenden beschriebene Maßnahme dafür Sorge getragen wird, dass während der gesamten Hydrierung ein Peroxidgehalt von höchstens 0,1 Mol/l, vorzugsweise von höchstens 0,05 Mol/l, und insbesondere von höchstens 0,02 Mol/l eingestellt und aufrecht erhalten wird. Dazu wird in einem Hydrierreaktor eine Suspension des Katalysators in dem bei der Ozonolyse als Lösungsmittel verwendeten Alkohol vorgelegt und die bei der Ozonisierung erhaltene Lösung mittels einer regelbaren Dosiervorrichtung kontinuierlich eingespeist. Bei der Zugabe der Ozonolyselösung zu Beginn und im Verlaufe der Hydrierung ist selbstverständlich darauf zu achten, dass durch die zugeführte Menge der peroxidhältigen Ozonolyseprodukte der oben angegebene Peroxidgehalt im Hydrierreaktor nicht überschritten wird.

Durch die geringe Konzentration an peroxidhältiger Ozonolyselösung im Reaktionsmedium während des eigentlichen Hydriervorganges infolge der kontinuierlichen Einspeisung ist eine rasche Reduktion gewährleistet. Auf diese Weise wird eine Vergiftung des Katalysators und der damit verbundene Aktivitätsverlust verhindert. Im Gesamten gesehen kann aber durch die kontinuierliche Zugabe eine große Menge an Ozonolyseprodukten in einem verhältnismäßig kleinem Volumen reduziert werden. wodurch hohe Konzentrationen an entsprechendem HMP entstehen und Zeit, sowie Kosten bei der destillativen Entfernung der Lösungsmittel gespart werden.

Als Katalysatoren eignen sich die für Hydrierungen geeigneten Edelmetallkatalysatoren, die in Form von Pulverkontakten mit Trägermaterialien oder ohne Trägermaterial eingesetzt werden können. Bevorzugt werden Palladium- oder Platinkatalysatoren verwendet. Bei Pulverkontakten eignen sich als Trägermaterial beispielsweise Kohle, Aluminium. Silikagel oder Kieselgur. Die Ausbeuten sind an sich unabhängig von der eingesetzten Katalysatormenge jedoch empfiehlt es sich zur Erzielung einer ausreichenden Hydriergeschwindigkeit die genannten Katalysatoren in Edelmetallmengen von 0,1 bis 20 Gew.%, bevorzugt von 1 bis 15 Gew.% und besonders bevorzugt von 5 bis 10 Gew.% bezogen auf die jeweils pro Stunde eingespeiste Gesamtmenge der ozonisierten Vinylpyridine vorzulegen.

Nach Beendigung der Reduktion wird der Katalysator aus dem Reaktionsgemisch abgetrennt.

Beim erfindungsgemäßen Verfahren werden zur Reduktion der Ozonolyseprodukte äquivalente Mengen an Wasserstoff verbraucht. Die Menge an Wasserstoff, die bei der Hydrierung verwendet werden kann, reicht von einem Moläquivalent bis zu einem mehrfachen molaren Uberschuß. Bevorzugt werden 1,3 bis 2, 5 Moläquivalent, besonders bevorzugt 1,8 bis 2.2 Molaquivalent an Wasserstoff eingesetzt.

Die Hydrierung erfolgt in vorteilhafter Weise unter praktisch drucklosen Bedingungen. Unter praktisch drucklosen Bedingungen sollen hier Drücke von 1 bis etwa 3 bar verstanden werden wie das in der Technik üblich ist, um das Eindringen von Luft in den Hydrierreaktor zu vernindern. Auf diese Weise ist die Reduktion der Ozonolyseprodukte technisch sehr einfach durchzuführen. Es ist aber auch möglich, die Hydrierung unter einem Druck bis zu 20 bar durchzuführen und dadurch die Hydriergeschwindigkeit zu steigern. Die Reduktion verlauft exotherm und wird gemäß einer bevorzugten Ausführungsform bei Temperaturen von 20 bis 60 °C, insbesondere bei Temperaturen im Bereich von 35 bis 50 °C ausgefuhrt.

Nach vollendeter Hydrierung wird die Reaktionslösung vom Katalysator abgetrennt und bevorzugt beispielsweise am Rotationsverdampfer, eingeengt, wobei der als Lösungsmittel verwendete Alkohol als Alkohol/Wasser-Gemisch abdestilliert wird. Auch das bei der Umsetzung der Vinylpyridine zu dem entsprechenden HMP entstehende Nebenprodukt Formaldehyd wird dabei größtenteils aus der Reaktionslösung entfernt. Um den Alkohol zu recyklieren, kann der Formaldehyd beispielsweise mittels saurem lonentauscher in Formaldehyddialkylacetal überführt werden, destillativ vom Alkohol abgetrennt und anschließend verbrannt werden.

Nach dem Einengen wird die verbleibende Reaktionslösung sodann mit einer geeigneten Base, wie etwa NaOH, KOH, CaOH u.s.w, bevorzugt mit NaOH in Kombination mit Ammoniakwasser, bevorzugt 25%ig, versetzt, sodass ein pH-Wert von 10 bis 14, bevorzugt von 11-13 eingestellt wird. Bei diesem exothermen Vorgang wird das Nebenprodukt Picolinsäurealkylester verseift und der restliche Formaldehyd in Urotropin überführt.

Die nach dem Einengen verbleibende Reaktionslösung kann jedoch auch nur mit einer geeigneten Base, wie etwa NaOH, KOH, CaOH u.s.w, bevorzugt 20 bis 50%ige NaOH, ohne Ammoniakwasser, wiederum bis zum Erreichen eines pH-Wertes von 10 bis 14, bevorzugt von 11-13 , versetzt und auf 60 bis 110°C, bevorzugt 75 bis 85°C erwärmt werden. In diesem Fall wird das Nebenprodukt Picolinsäurealkylester wiederum verseift und der restliche Formaldehyd in Formiat und Methanol überführt.

Als bevorzugte Ausführungsform wird die Hydrierlösung am Rotationsverdampfer in eine basische Vorlage, wie etwa eine NaOH-, KOH-, CaOH-Vorlage, bevorzugt in eine 20-50%ige NaOH-Vorlage eingesaugt und gleichzeitig Alkohol und Wasser abdestilliert, wodurch wiederum das Nebenprodukt Picolinsäurealkylester verseift und der restliche Formaldehyd in Formiat und Methanol überführt wird, jedoch so gut wie kein Formaldehyd im Destillat nachgewiesen werden kann. Auch hier ist der pH-Wert zwischen 10 und 14.

In allen drei Fällen wird der stark basische Sumpf , nachdem ein Wassergehalt von 30 bis 70%, bevorzugt von 40 bis 60% und besonders bevorzugt von 45 bis 55%, eingestellt wurde, mit einem organischen Lösungsmittel bei einer Temperatur von 15-50°C, bevorzugt von 20-45°C extrahiert. Geeignete Lösungsmittel sind hierbei übliche unter den Reaktionsbedingungen inerte Extraktionsmittel, wie etwa Toluol, Ether u.s.w. Bevorzugt werden Ether verwendet, besonders bevorzugt Methyl-t.-butylether (MTBE).

Um eine vollständige Extraktion zu gewährleisten, sind in Abhängigkeit vom verwendeten Lösungsmittel und der Löslichkeit des HMPs in dem Lösungsmittel mehrere Extraktionen notwendig. Die gesammelten organischen Phasen werden sodann eingeengt und der Rückstand bei 10 bis 200 mbar, bevorzugt bei 30 bis 100 mbar, besonders bevorzugt bei 40 bis 60 mbar , und maximal notwendiger Sumpftemperatur destilliert.

Bei der Destillation werden dabei bevorzugt etwa 5% Vorlauf und 5% Sumpf abgetrennt.

Ein Vorteil bei der erfindungsgemäßen Aufarbeitung ist, dass insbesondere bei der bevorzugten Ausführungsform kein Formaldehyd verbrannt werden muss und das bei einer eventuell unvollständigen Hydrierung der entsprechende durch die Ozonolyse erhaltene Pyridinaldehyd durch den im Überschuss vorhandenen Formaldehyd zum größten Teil (über 70%) durch eine gekreuzte Cannizzaro-Reaktion in das entsprechende Hydroxymethylpyridin überführt wird.

Ein weiterer Vorteil ist, dass die durch den Ozonolyse/Reduzierungs-Mechanismus resultierenden Nebenprodukte, wie etwa Pyridincarbonsäure, Pyridincarbonsäureester, Pyridinaldehyd und Formaldehyd leicht abgetrennt werden können und die Endprodukte somit in Ausbeuten von bis zu 80% und einem Gehalt von über 99,5 Gew.% erhalten werden.

### Beispiel 1: Herstellung von 2-Hydroxymethylpyridin

Eine 0.5 molare Lösung von 2-Vinylpyridin in Methanoi wurde bei -15°C mit Ozon in einer Konzentration von 50g/m³h ozonisiert und anschließend die so erhaltene Lösung über ein Dosiergefäß in einen Hydrierreaktor, in dem eine Suspension von 5% Pd/C-Katalysator in Methanol vorgelegt war und der mit Wasserstoff gefüllt war, in einer solchen Dosierung eingespeist, dass der Peroxidgehalt 0,02 Mol/l nicht überstieg. Unter kräftigem Rühren und Wasserstoffzugabe wurde bis zur negativen Peroxidprobe hydriert, wobei die Temperatur auf 45°C gehalten wurde. Nach beendeter Hydrierung wurde der Katalysator abgetrennt.

### Schritt b)

Die so erhaltene Hydrierlösung wurde kontinuierlich am Rotationsverdampfer in eine 40%ige Natronlaugevorlage die einen pH-Wert über 12 aufwies, eingesaugt und gleichzeitig bei 80°C und leichtem Vakuum Methanol/Wasser abdestilliert. Bei diesem 3,5 stündigem Vorgang wurde der Picolinsäuremethylester verseift und der Formaldehyd in Natriumformiat und Methanol überführt. Im Destillat konnte durch Oximtitration so gut wie kein Formaldehyd nachgewiesen werden ( weniger als 50 mg/l Methanol).

### Schritt c)

Der stark basische Sumpf wurde, nachdem ein Wassergehalt von ca. 50% eingestellt wurde, bei Raumtemperatur mit MTBE extrahiert, wobei in einem Liter MTBE 40g 2-Hydroxymethylpyridin löslich waren. Um eine vollständige Extraktion zu gewährleisten, waren 10 Extraktionen notwendig. Pro kg eingesetztem 2-Vinylpyridin wurden ca. 1,7 kg Extraktionssumpf (ca. 65% Wassergehalt) abgetrennt.

Die gesammelten organischen Phasen wurden am Rotationsverdampfer bei 60°C und leichtem Vakuum eingeengt und der Rückstand bei 50 mbar und maximal der notwendigen Sumpftemperatur von 140°C abdestilliert. Bei der Destillation wurden ca. 5% Vorlauf, enthaltend u.a. Ethylpyridin, Wasser u.s.w, und 5% Sumpf abgetrennt.

Der Gehalt der farblosen Hauptfraktion betrug > 99% und der Formaldehydgehalt < 100ppm.

Die Ausbeute an 2-Hydroxymethylpyridin betrug 67% bezogen auf eingesetztes 2-Vinylpyridin.

### Beispiel 2:

### Ozonolyse und Hydrierung wurden analog Beispiel 1 durchgeführt.

### Schritt b)

Die so erhaltene Hydrierlösung wurde kontinuierlich am Rotationsverdampfer eingesaugt und gleichzeitig bei 80°C und leichtem Vakuum Methanol/Wasser/Formaldehyd abdestilliert. Bei diesem 3,5 stündigem Vorgang wurde der Formaldehyd zu 90% abdestilliert. Anschließend wurde durch Zugabe von 40%iger Natronlauge im Überschuss der Picolinsäuremethylester verseift und der restliche Formaldehyd in Natriumformiat und Methanol bei 80°C innerhalb von 2 Stunden überführt.

### Schritt c)

Der stark basische Sumpf wurde, nachdem ein Wassergehalt von ca. 50% eingestellt wurde, bei Raumtemperatur mit MTBE extrahiert wobei in einem Liter MTBE 40g 2-Hydroxymethylpyridin löslich waren. Um eine vollständige Extraktion zu gewährleisten waren 10 Extraktionen notwendig.

Die gesammelten organischen Phasen wurden am Rotationsverdampfer bei 60°C und leichtem Vakuum eingeengt und der Rückstand bei 50 mbar und maximal der notwendigen Sumpftemperatur von 150°C abdestilliert. Bei der Destillation wurden ca. 5% Vorlauf, enthaltend u.a. Ethylpyridin. Wasser u.s.w. und 5% Sumpf abgetrennt.

Der Gehalt der farblosen Hauptfraktion betrug > 99% und der Formaldehydgehalt < 100ppm.

Die Ausbeute an 2-Hydroxymethylpyridin betrug 76% bezogen auf eingesetztes 2-Vinylpyridin.

## Patentansprüche

1. Verfahren zur Herstellung von Hydroxymethylpyridinen, **dadurch gekennzeichnet, dass**
a) das entsprechende Vinylpyridin in einem Alkohol als Lösungmittel ozonisiert und die dabei entstehende peroxidhaltige Lösung in Gegenwart eines Hydrierkatalysators hydriert wird,
b) die Hydrierlösung durch Zugabe einer Base auf einen pH-Wert von 10-bis14 gestellt und der Alkohol abdestilliert wird, worauf
c) das gewünschte Hydroxymethylpyridin durch Extraktion mit einem organischem Lösungsmittel und nachfolgender destillativer Aufreinigung in hoher Reinheit isoliert wird.

2. Verfahren nach Anspruch 1, **dadurch gekennzeichnet, dass** 2-, 3- oder 4-Hydroxymethylpyridin, 6-Methyl-2-hydroxymethylpyridin oder 2,6-Di(Hydroxymethyl)-pyridin hergestellt werden.

3. Verfahren nach Anspruch 1, **dadurch gekennzeichnet, dass** als Alkohol ein aliphatischer C₁-C₄-Alkohol eingesetzt wird.

4. Verfahren nach Anspruch 1, **dadurch gekennzeichnet, dass** die Hydrierung bei 1-3 bar durchgeführt wird.

5. Verfahren nach Anspruch 1, **dadurch gekennzeichnet, dass** nach der Hydrierung der Katalysator abgetrennt, die Reaktionslösung durch Abdestillieren eines Alkohol/Wasser-Gemisches eingeengt und anschließend durch Zugabe einer Base auf einen pH-Wert von 10-14 gestellt wird.

6. Verfahren nach Anspruch 1, **dadurch gekennzeichnet, dass** nach der Hydrierung der Katalysator abgetrennt, die Reaktionslösung in eine basische Vorlage eingebracht und gleichzeitig Alkohol und Wasser abdestilliert wird.

7. Verfahren nach Anspruch 1, **dadurch gekennzeichnet, dass** in Schritt c) der durch Abdestillieren des Alkohols und Zugabe einer Base erhaltene stark basische Sumpf auf einen Wassergehalt von 30 bis 70% gestellt wird und anschließend bei einer Temperatur von 15 bis 50°C das entsprechende Hydroxymethylpyridin mit einem unter den Reaktionsbedingungen inerten, organischen Lösungsmittel aus dem Sumpf extrahiert wird.

8. Verfahren nach Anspruch 1, **dadurch gekennzeichnet, dass** in Schritt c) als organisches Lösungsmittel zur Extraktion Ether verwendet werden.

9. Verfahren nach Anspruch 1, **dadurch gekennzeichnet, dass** die destillative Aufreinigung der durch die Extraktion erhaltenen organischen Phasen bei 10 bis 200 mbar erfolgt.

## Claims

1. A process for preparing hydroxymethylpyridines which comprises
a) subjecting the corresponding vinylpyridine to ozonolysis in an alcohol solvent and hydrogenating the resulting peroxidic solution in the presence of a hydrogenation catalyst,
b) adjusting the hydrogenation solution by addition of a base to a pH of from 10 to 14 and distilling off the alcohol, and then
c) isolating the desired hydroxymethylpyridine in high purity by extraction with an organic solvent and subsequent distillative purification.

2. The process according to claim 1, wherein 2-, 3- or 4-hydroxymethylpyridine, 6-methyl-2-hydroxymethylpyridine or 2,6-di(hydroxymethyl)pyridine are prepared.

3. The process according to claim 1, wherein the alcohol used is an aliphatic C₁-C₄-alcohol.

4. The process according to claim 1, wherein the hydrogenation is carried out at from 1 to 3 bar.

5. The process according to claim 1, wherein the catalyst is separated off after the hydrogenation, the reaction solution is concentrated by distilling off an alcohol-water mixture and the pH is set to 10 to 14 by addition of a base.

6. The process according to claim 1 wherein the catalyst is separated off after the hydrogenation, the reaction solution is introduced into a basic initial charge and water and alcohol are simultaneously distilled off.

7. The process according to claim 1, wherein the water content of the strongly basic bottom product obtained in step c) by distilling off the alcohol and adding a base is set from 30 to 70% and the resulting hydroxymethylpyridine is then extracted from the bottom product using an organic solvent that is inert under the reaction conditions at a temperature of from 15 to 50°C.

8. The process as claimed in claim 1, wherein the organic solvent used for extraction in step c) is ether.

9. The process as claimed in claim 1, wherein the distillative purification of the organic phases obtained by extraction is carried out at from 10 to 200 mbar.

## Revendications

1. Procédé de fabrication d'hydroxyméthylpyridines **caractérisé en ce que**
a) la vinylpyridine correspondante est ozonisée dans un alcool servant de solvant et que la solution de peroxyde obtenue est hydrogénée en présence d'un catalyseur d'hydrogénation,
b) la solution d'hydrogénation est distillée par ajout d'une base à un pH ajusté de 10 à 14 et d'un alcool,
c) l'hydroxyméthylpyridine souhaitée est isolée par extraction avec un solvant organique et purification, par distillation consécutive, à un degré élevé de pureté.

2. Procédé selon la revendication 1, **caractérisé en ce que** des 2-, 3- ou 4-hydroxyméthylpyridines, de la 6-méthyl-2-hydroxyméthylpyridine ou de la 2,6-di(hydroxyméthyl)-pyridine sont produites.

3. Procédé selon la revendication 1, **caractérisé en ce que** l'alcool utilisé en un alcool aliphatique en C₁-C₄.

4. Procédé selon la revendication 1, **caractérisé en ce que** l'hydrogénation est effectuée à une pression de 1 à 3 bars.

5. Procédé selon la revendication 1, **caractérisé en ce qu'**après l'hydrogénation, le catalyseur étant séparé, la solution réactionnelle est concentrée par distillation d'un mélange alcool/eau et ensuite est ajustée par ajout d'une base à un pH de 10 à 14.

6. Procédé selon la revendication 1, **caractérisé en ce qu'**après l'hydrogénation, le catalyseur étant séparé, la solution réactionnelle est placée dans une composition basique et en même temps séparée par distillation en alcool et eau.

7. Procédé selon la revendication 1, **caractérisé en ce que** dans l'étape c), le milieu fortement basique obtenu par distillation de l'alcool et ajout d'une base est ajusté à une teneur en eau de 30 à 70 % et ensuite, à une température de 15 à 50° C, est extraite du milieu l'hydroxyméthylpyridine correspondante avec un solvant organique inerte dans les conditions de réaction.

8. Procédé selon la revendication 1, **caractérisé en ce que** de l'éther est utilisé dans l'étape c) comme solvant organique pour extraction.

9. Procédé selon la revendication 1, **caractérisé en ce que** la purification par distillation des phases organiques obtenues par extraction s'effectue à une pression de 10 à 200 mbar.
